(19) Europäisches Patentamt
European Patent Office
Office européen des brevets

(11) Veröffentlichungsnummer: **0 311 100**
**A2**

# (12) EUROPÄISCHE PATENTANMELDUNG

(21) Anmeldenummer: **88116655.7**

(51) Int. Cl.$^4$: **C07D 405/04 , A61K 31/505**

(22) Anmeldetag: **07.10.88**

(30) Priorität: **09.10.87 US 107221**

(43) Veröffentlichungstag der Anmeldung:
**12.04.89 Patentblatt 89/15**

(84) Benannte Vertragsstaaten:
**AT BE CH DE ES FR GB GR IT LI LU NL SE**

(71) Anmelder: **F. HOFFMANN-LA ROCHE & CO. Aktiengesellschaft**

**CH-4002 Basel(CH)**

(72) Erfinder: **Partridge, John Joseph**
**620 Airport Road**
**Chapel Hill No. Carolina 27514(US)**
Erfinder: **Tam, Steve**
**13 Evergreen Road**
**West Caldwell, N.J. 07006(US)**

(74) Vertreter: **Lederer, Franz, Dr. et al**
**Van der Werth, Lederer & Riederer**
**Patentanwälte Lucile-Grahn-Strasse 22**
**D-8000 München 80(DE)**

## (54) Neue Dideoxycytidin-Derivate, ihre Herstellung und Verwendung für Pharmazeutika.

(57) Verbindung der Formel

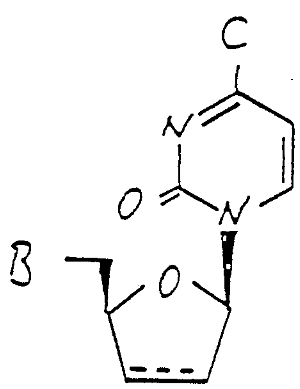

I

worin die punktierte Bindung fakultativ ist, B OH oder OC(O)R; C NHC(O)$R^1$ oder N=CH-N$R^2R^3$; R, $R^1$, $R^2$ und $R^3$ unabhängig voneinander geradkettiges oder verzweigtes Alkyl, Alkoxyalkyl, Aralkyl, Aryloxyalkyl oder Aryl darstellen, sind wirksam gegen retrovirale Infektionen, insbesondere AIDS.



Xerox Copy Centre

VERBINDUNG 1 a

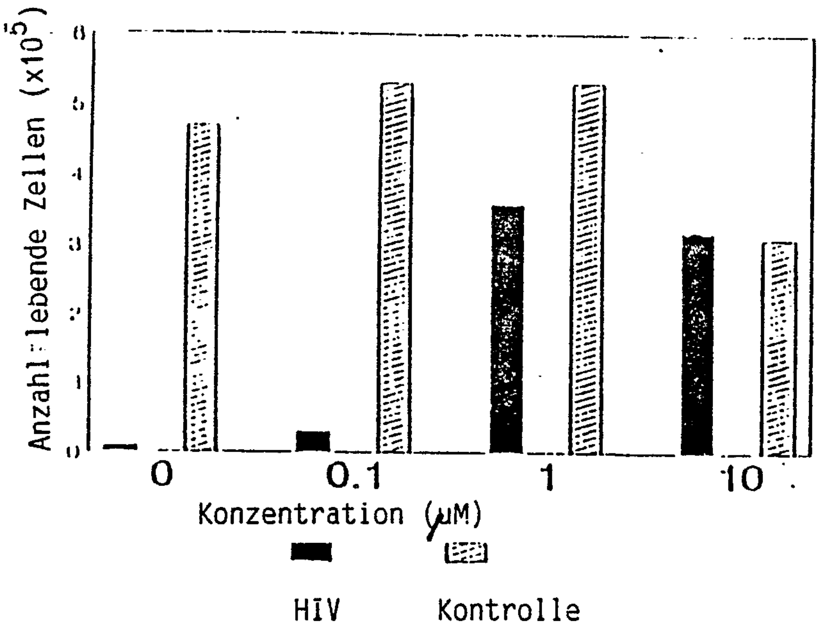


Figur 1

POOR QUALITY

## Neue Dideoxycytidin-Derivate, ihre Hestellung und Verwendung für Pharmazeutika

Die vorliegende Erfindung betrifft neue Dideoxycytidin-Derivate. Diese Verbindungen weisen grössere Stabilität und Lipophilität als bekannte Nucleosidderivate auf und durchdringen die Blut-Hirnschranke wirksamer.

Die vorliegende Erfindung betrifft weiterhin alle neuen Zwischenprodukte, die in der Synthese der erfindungsgemässen Verbindungen verwendet werden; ein Verfahren zur Herstellung der genannten Dideoxycytidin-Derivate und die Anwendung der erfindungsgemässen Verbindungen zur Herstellung von pharmazeutischen Präparaten zur Behandlung retroviraler Infektionen, insbesondere Aids (HIV).

Insbesondere betrifft die vorliegende Erfindung Verbindungen der Formel

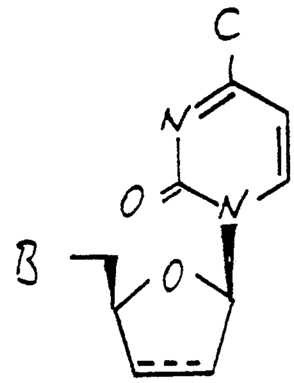

I

worin die punktierte Bindung fakultativ ist, B OH oder OC(O)R; C $NHC(O)R^1$ oder $N=CH-NR^2R^3$; R, $R^1$, $R^2$ und $R^3$ unabhängig voneinander geradkettiges oder verzweigtes Alkyl, Alkoxyalkyl (z.B. Methoxymethyl), Aralkyl (z.B. Benzyl), Aryloxyalkyl (z.B. Phenoxymethyl), Aryl (z.B. Phenyl, gewünschtenfalls substituiert durch Halogen, $C_{1-4}$-Alkyl oder $C_{1-4}$-Alkoxy) darstellen. Alkyl bedeutet insbesondere Alkylgruppen mit 1-7 C-Atomen.

Bevorzugte Verbindungen der Formel I sind diejenigen, in denen die punktierte Bindung abwesend ist. Weiterhin sind Verbindungen der Formel I bevorzugt, in denen B OC(O)R und R Methyl, Aethyl, Propyl, Isopropyl, n-Pentadecyl, n-Heptadecyl, Phenyl oder Benzyl darstellt.

Weiterhin sind Verbindungen der Formel I bevorzugt, in denen C $NHC(O)R^1$ und $R^1$ Methyl, Aethyl, Propyl, Isopropyl, n-Pentadecyl, n-Heptadecyl, Phenyl oder Benzyl darstellen.

Besonderes bevozugt sind Verbindungen der Formel I, in denen B OH ist, wie N-Butanoyl-2′,3′-dideoxycytidin.

Das erfindungsgemässe Verfahren zur Herstellung der Verbindung der Formel I ist dadurch gekennzeichnet, dass man

a) eine Verbindung der Formel

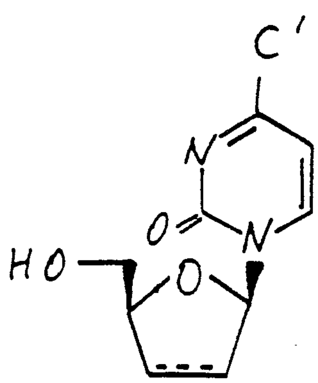

II

worin die punktierte Bindung fakultativ ist und C′ $NH_2$ oder $N=CH-NR^2,R^3$ darstellt,

mit einem Anhydrid der Formel $(RCO)_2O$ oder einem Halogenid der Formel RCOX, worin R die obige Bedeutung hat und X Halogen ist, umsetzt oder

b) eine Verbindung der Formel II, worin C′ $NH_2$ ist, mit einer Verbindung der Formel

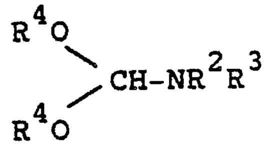

worin $R^4$ $C_{1-4}$-Alkyl und $R^2$ und $R^3$ die obige Bedeutung haben.
umsetzt und gewünschtenfalls die in einer erhaltenen Verbindung der Formel I vorhandene punktierte Bindung hydriert und/oder eine Estergruppe B oder eine Acylamido oder Schiff-Base-Gruppe C in einer erhaltenen Verbindung der Formel I selektiv hydrolysiert.

Beispiele von Anhydriden der Formel $(RCO)_2O$ sind aromatische Anhydride wie Benzoesäureanhydrid, Aralkancarbonsäureanhydride wie Phenylessigsäureanhydrid und Alkancarbonsäureanhydride wie Acetanhydrid, Propionsäureanhydrid, 2-Methylpropionsäureanhydrid, Butansäureanhydrid, Palmitinsäureanhydrid und Stearinsäureanhydrid. Beispiele von Halo geniden der Formel RCOX sind aromatische Säurehalogenide wie Benzoylchlorid, Aralkanoylhalogenide wie Phenylacetylchlorid und Alkanoylhalogenide wie Acetylchlorid. Vorzugsweise wird eine Verbindung der Formel II mit einem Anhydrid der Formel $(RCO)_2O$ umgesetzt. Gewünschtenfalls wird die Umsetzung einer Verbindung der Formel II mit einem Anhydrid $(RCO)_2O$ oder einem Halogenid RCOX in Gegenwart eines Kondensationsmittels, z.B. einer organischen Base wie Pyridin, Dimethylaminopyridin oder Trimethylamin durchgeführt. Erforderlichenfalls kann ein organisches Lösungsmittel, z.B. Dimethylformamid anwesend sein. Alternativ kann die Base, z.B. Pyridin, als Lösungsmittel verwendet werden. Wenn eine Ausgangsverbindung der Formel II verwendet wird, worin C′ $NH_2$ ist, wird eine Verbindung der Formel I erhalten, worin B $-O(CO)R^1$ und C $-NH(CO)R^1$ ist, d.h. die Acylierung tritt sowohl an der 5′-Hydroxygruppe als auch an der Aminogruppe ein. Wenn die Acylierung in Gegenwart eines alkoholischen Lösungsmittels wie Aethanol vorgenommen wird, wird nur die $NH_2$-Gruppe acyliert, sodass Verbindungen der Formel I erhalten werden, worin B OH und C $-NH(CO)R^1$ ist. Verbindungen der Formel I, worin B -O(CO)R und C $NH_2$ ist, können durch Hydrolyse einer Verbindung der Formel I erhalten werden, worin B -O(CO)R und C $-N=CH-NR^2R^3$ ist. Die letzteren Verbindungen können wiederum durch Umsetzung von 2′,3′-Didehydro-2′,3′-dideoxycytidin oder 2′,3′-Dideoxycytidin (d.h. eine Verbindung der Formel II, worin C′ $NH_2$ ist) mit einer Verbindung der Formel $(R^4O)_2CHNR^2R^3$, z.B. N,N-Dimethylformamid-dimethylacetal erhalten werden.

Verbindungen der Formel I, worin die punktiere Bindung anwesent ist, können mittels bekannter Methoden hydriert werden, z.B. durch katalytische Hydrierung mit Edelmetallkatalysatoren, wobei entsprechende Verbindungen der Formel I erhalten werden, in denen die punktierte Bindung abwesend ist.

Schliesslich werden annähernd gleiche Mengen des 5′-Hydroxyesters und der N-Amidderivate erhalten, wenn eine 5′-Hydroxyester-N-amidverbindung mit Ammoniak/Wasser/Methanol oder Methanol/Wasser/Triäthylamin behandelt wird.

Wie oben erwähnt, zeigen die erfindungsgemässen Verbindungen grössere Stabilität und Lipophilität als bekannte Nucleotidderivate. In der nachstehenden Tabelle I sind für ausgewählte erfindungsgemässe Verbindungen die gemessenen Verteilungskoeffizienten angegeben, die wiederum ein Anzeichen für die Lipophilität der Verbindung darstellt.

Tabelle I

| Scheinbarer Verteilungskoeffizient zwischen n-Octanol und Phosphatpuffer pH 7,4* | |
|---|---|
| Verbindung | Verteilungskoeffizient |
| N-Acetyl 2′,3′-didehydro-2′,3′-dideoxycytidin 5′-acetat | 0.39 |
| N-Acetyl 2′,3′-dideoxycytidin 5′-acetat | 0.62 |
| N-Acetyl 2′,3′-dideoxycytidin | 0.22 |
| 2′,3′-Dideoxycytidin 5′-acetat | 0.14 |
| N-Propanoyl 2′,3′-dideoxycytidin 5′-propanoat | 6.8 |
| N-Propanoyl 2′,3′-dideoxycytidin | 0.83 |
| N-(2-Methylpropanoyl) 2′,3′-dideoxycytidin 5′-(2-methylpropanoat) | 58.0 |
| N-(2-Methylpropanoyl) 2′,3′-dideoxycytidin | 2.38 |
| N-Butanoyl 2′,3′-dideoxycytidin 5′-butanoat | 70.0 |
| N-Butanoyl 2′,3′-dideoxycytidin | 2.67 |
| N-Palmitoyl 2′,3′-dideoxycytidin 5′-palmitoat | > 1000 |
| N-Benzoyl 2′,3′-dideoxycytidin | 15.0 |
| 2′,3′-Didehydro-2′,3′-dideoxycytidin | 0.03 |
| 2′,3′-Dideoxycytidin | 0.05 |

* Der scheinbare Verteilungskoeffizient zwischen n-Octanol und wässrigem Phosphatpuffer pH 7,4 (pH des menschlichen Bluts) wurde nach der Methode von Hansch et al., Chem. Rev. 71, 525 (1971) bestimmt.

Die Stabilität ausgewählter erfindungsgemässer Verbindungen in Methanol/Wasser oder menschlichem Blutplasma wurde wie nachstehend angegeben geprüft:

Die folgenden Verbindungen wurden auf Stabilität im menschlichen Plasma geprüft:

| Verbindung | Nr. |
|---|---|
| N-Propanoyl 2′,3′-dideoxycytidin 5′-propanoat | 1a |
| N-(2-Methylpropanoyl) 2′,3′-dideoxycytidin 5′-(2-methylpropanoat) | 1b |
| N-Butanoyl 2′,3′-dideoxycytidin 5′-butanoat | 1c |
| N-Propanoyl 2′,3′-dideoxycytidin | 2a |
| N-(2-Methylpropanoyl) 2′,3′-dideoxycytidin | 2b |
| N-Butanoyl 2′,3′-dideoxycytidin | 2c |
| 2′,3′-Dideoxycytidin | 4 |

Zunächst wurden die HPLC-Bedingungen für die Probenmessung bestimmt, d.h. eine Revers-Phasen C-18-Säule mit einem Lösungsmittelsystem mit variierenden Verhältnissen von Methanol: 0,0001M Natriumphosphat, pH 6,8.

Danach wurde die maximale UV-Absorption für jede Probe und die grösste Stabilität unter Laborbedingungen für jede Probe in Methanol:Wasser bestimmt. Danach wurde die Stabilität im menschlichen Plasma untersucht.

Das im Experiment verwendete menschliche Plasma wurde bis zur Verwendung eingefroren.

Die HPLC-Apparatur war ein Gradientsystem bestehend aus einem Modell 1601 Gradient Master, einer Constametric I Pumpe und einer Constametric III Pumpe. Weiterhin wurden ein LDC-Spectromometer III als Detektor und ein Waters 712B Wisp Autoinjektor verwendet. Allen Proben wurde eine ODS-3 RAC II, 5 μ, 15 cm x 4,0 mm ID-Säule (Whatmann, Clifton, N.J.) verwendet. Das Lösungsmittelsystem bestand aus Methanol: 0,001M $NaH_2PO_4$ (pH 6,8) in den folgenden Proportionen:

1b, 2b - 50:50;

1a, 2a - 43:57;

1c, 2c - 50:50.

Jede Verbindung wurde in ein konisches 15 ml Teströhrchen eingewogen und in DMSO gelöst, so dass eine Vorratslösung von 10 mg/ml erhalten wurde. Die Proben für die Standardkurve wurden durch Verdünnen der DMSO-Vorratslösung mit der mobilen Phasen wie folgt hergestellt:

| | |
|---|---|
| Für 100 µg/ml | Verdünnen von 0,04 ml Grundlösung auf 4 ml (Lösung 1) |
| 50 µg/ml | Verdünnen von 2 ml Lösung 1 auf 4 ml (Lösung 2) |
| 10 µg/ml | Verdünnen von 0,8 ml Lösung 2 auf 4 ml (Lösung 3) |

Für die Untersuchungen wurden die Proben mit Plasma verdünnt (0,07 ml --- 7 ml), 0,4 ml Portionen wurden entnommen, filtriert (0,22 µ, Gelman) und die Zeiten wurden notiert (0, 0,5, 1, 2, 4 und 24 Stunden).

Die chromatographischen Daten wurden mit einem Nelson 3000 Datasystem gesammelt. Peakhöhen der Kalibrierungs-Standards jeder Verbindung wurden gemessen und gegen bekannte Konzentrationen aufgetragen. Eine lineare Kurve der kleinsten Quadrate (ungewichtet) wurde berechnet und alle Probenkonzentrationen wurden aus dieser Kurve rückgerechnet. Die Resultate sind in den Tabellen II-VIII zusammengestellt.

Tabelle II

| Stabilität in Methanol/Wasser | | | | | |
|---|---|---|---|---|---|
| Verbindung Nr. | Zeit (Tage) | % N, O-Diacyl (1a,1b,1c) | % N-Acyl (2a,2b, 2c) | % O-Acyl- (3a,3b,3c) | % ddC 4 |
| 1a | 0 | 100 | - | - | - |
| | 14 | 43 | 18 | 27 | - |
| 2a | 0 | - | 100 | - | - |
| | 14 | - | 56 | - | 28 |
| 1b | 0 | 100 | - | - | - |
| | 14 | 73 | 8 | 17 | 2 |
| 2b | 0 | - | 100 | - | - |
| | 14 | - | 83 | 17 | 2 |
| 1c | 0 | 100 | - | - | - |
| | 14 | 43 | 20 | 32 | 25 |
| 2c | 0 | - | 100 | - | - |
| | 14 | - | 76 | - | 20 |

Tabelle III

| Stabilität in Humanplasma bei 37° C (µg/ml) | | | | | |
|---|---|---|---|---|---|
| Verbindung Nr. | Zeit (Tage) | % N, O-Diacyl (1a) | % N-Acyl (2a) | % O-Acyl | % ddC (4) |
| 1a | 0 | 86 | 6 | - | 1 |
| | 0,5 | 70 | 22 | - | 2 |
| | 1 | 40 | 42 | - | 1 |
| | 2 | 17 | 79 | - | 1 |
| | 4 | 10 | 91 | - | 1 |
| | 24 | 1 | 81 | - | 3 |

Tabelle IV

| Stabilität in Humanplasma bei 37° C (µg/ml) | | | |
|---|---|---|---|
| Verbindung Nr. | Zeit (Tage) | % N-Acyl (2a) | % ddC (4) |
| 2a | 0 | 97 | 1 |
| | 0,5 | 87 | 1 |
| | 1 | 85 | 2 |
| | 2 | 96 | - |
| | 4 | 99 | - |
| | 24 | 80 | 3 |

Tabelle V

| Stabilität in Humanplasma bei 37° C (µg/ml) | | | | | |
|---|---|---|---|---|---|
| Verbindung Nr. | Zeit (Tage) | % N, O-Diacyl (1b) | % N-Acyl (2b) | % O-Acyl (3b) | % ddC (4) |
| 1b | 0 | 95 | 3 | - | - |
| | 0,5 | 83 | 8 | - | - |
| | 1 | 38 | 49 | - | - |
| | 2 | 9 | 60 | - | - |
| | 4 | 4 | 72 | - | - |
| | 24 | 3 | 88 | - | 5 |

Tabelle VI

| Stabilität in Humanplasma bei 37° C (µg/ml) | | | |
|---|---|---|---|
| Verbindung Nr. | Zeit (Tage) | % N-Acyl (2b) | % ddC (4) |
| 2b | 0 | 83 | - |
| | 0,5 | 88 | - |
| | 1 | 61 | - |
| | 2 | 78 | - |
| | 4 | 89 | - |
| | 24 | 92 | - |

Tabelle VII

| Stabilität in Humanplasma bei 37° C (µg/ml) | | | | | |
|---|---|---|---|---|---|
| Verbindung Nr. | Zeit (Tage) | % N, O-Diacyl (1c) | % N-Acyl (2c) | % O-Acyl (3c) | % ddC (4) |
| 1c | 0 | 64 | 4 | - | - |
| | 0,5 | 23 | 7 | - | - |
| | 1 | 10 | 71 | - | - |
| | 2 | 7 | 74 | - | - |
| | 4 | 6 | 72 | - | - |
| | 24 | 6 | 68 | - | 5 |

Tabelle VIII

| Stabilität in Humanplasma bei 37° C (µg/ml) | | | |
|---|---|---|---|
| Verbindung Nr. | Zeit (Tage) | % N-Acyl (2c) | % ddC (4) |
| 2c | 0 | 101 | - |
| | 0,5 | 95 | - |
| | 1 | 92 | - |
| | 2 | 90 | - |
| | 4 | 85 | - |
| | 24 | 77 | - |

Ausgewählte erfindungsgemässe Verbindungen wurden in dem von Mitsuya et al., Science, Vol. 226, pp. 172-174 (1984) beschriebenen Test auf Hemmung des HIV-cytopathischen Effekts geprüft.

| | |
|---|---|
| Konzentration der verwendeten Verbindung: | 0,1, 1, 10, 20, 100 µM |
| Verwendetes Verdünnungsmittel: | Phosphat gepuffterte Kochsalzlösung |
| Verwendete Zellen: | ATH8 Zellen (2x$10^5$ pro Probe) |
| Verwendetes Viruspräparat: | HTLV-$III_B$ (2000 Viruspartikel/Zelle) |

Die Resultate sind in den Figuren 1-5 dargestellt. Schwarze Säulen stellen die mit der Verbindung behandelten überlebenden Zellen nach Zusatz von HTLV-III dar. Helle Säulen stellen mit der Verbindung behandelten Zellen in Abwesenheit des Virus dar.

Die erfindungsgemässen Verbindungen können in jeder geeigneten Weise verabreicht werden, z.B. oral oder intravenös. Es kann auch ein alternierendes Dosierungsschema angewandt werden. Die Dosierung kann auch in Kombination mit anderen antiviralen Therapeutika, z.B. mit 3′-Azido-3-deoxythymidin (AZT) zusammen erfolgen.

Obwohl es möglich ist, die Verbindungen der Formel I als solche zu verabreichen, werden sie vorzugsweise in Form pharmazeutischer Präparate angewandt. Die erfindungsgemässen Präparate enthalten mindestens eine Verbindung der Formel I mit einem oder mehreren annehmbaren Trägern oder gewünschtenfalls anderen Therapeutika.

Für die orale Verabreichung geeignete erfindungsgemässe Formulierungen können als diskrete Einheiten wie Kapseln oder Tabletten, die eine bestimmte Menge der Verbindung der Formel I enthalten, vorliegen oder als Pulver oder Granulate oder als Lösung oder Suspension in einer wässrigen oder nicht-wässrigen Flüssigkeit oder als Oel in Wasser-Emulsion oder Wasser in Oel-Emulsion. Die Verbindungen der Formel I können auch als Bolus oder Paste liegen.

Präparate die für die intravenöse Verabreichung geeignet sind, sind wässrige oder nicht-wässrige isotonische sterile Injektionslösungen, die Antioxidantien, Puffer, Bakteriostatika und Stoffe enthalten können, die die Formulierung mit dem Blut des Patienten isotonisch halten. Weitere Beispiele sind wässrige und nicht-wässrige sterile Suspensionen, die Suspendierungs- und Verdickungsmittel enthalten können.

Die nachstehenden Beispiele erläutern die Erfindung weiter. Alle Temperaturen sind in Celsiusgraden angegeben.

## Beispiel 1

Ein Gemisch von 0,021 g 2′,3′-Didehydro-2′,3′-dideoxycytidin, 0,25 ml Acetanhydrid und 1,0 ml Pyridin wurde über Nacht bei Raumtemperatur gerührt. Das Gemisch wurde unter vermindertem Druck zur Trockene eingedampft. Der Feststoff wurde mit 1 ml Tetrahydrofuran bei 0° behandelt, dann aus 20 ml heissem Tetrahydrofuran umkristallisiert. Man erhielt N-Acetyl 2′,3′-didehydro-2′,3′-dideoxycytidin 5′-acetat, Smp. >350°, $[\alpha]_D^{25}$ = +15,8° (c = 0,33, DMSO).

## Beispiel 2

In Analogie zu Beispiel 1 kann aus 2′,3′-Didehydro-2′,3′-dideoxycytidin mit Propionsäureanhydrid in Pyridin das N-Propanoyl 2′,3′-Didehydro-2′,3′-dideoxycytidin 5′-propanoat erhalten werden.

## Beispiel 3

In Analogie zu Beispiel 1 kann aus 2′,3′-Didehydro-2′,3′-dideoxycytidin und 2-Methylpropionsäureanhydrid in Pyridin das N-(2-Methylpropanoyl) 2′,3′-didehydro-2′,3′-dideoxycytidin 5′-(2-methylpropanoat) erhalten werden.

## Beispiel 4

In Analogie zu Beispiel 1 kann aus 2′,3′-Didehydro-2′,3′-dideoxycytidin mit Butansäureanhydrid in Pyridin das N-Butanoyl 2′,3′-didehydro-2′,3′-dideoxycytidin 5′-butanoat erhalten werden.

## Beispiel 5

In Analogie zu Beispiel 1 kann aus 2′,3′-Didehydro-2′,3′-dideoxycytidin und Palmitinsäureanhydrid in Pyridin das N-Palmitoyl 2′,3′-didehydro-2′,3′-dideoxycytidin 5′-palmitoat erhalten werden.

## Beispiel 6

In Analogie zu Beispiel 1 kann aus 2′,3′-Didehydro-2′,3′-dideoxycytidin und Stearinsäureanhydrid in Pyridin das N-Stearoyl 2′,3′-didehydro-2′,3′-dideoxycytidin 5′-stearat erhalten werden.

## Beispiel 7

In Analogie zu Beispiel 1 kann aus 2′,3′-Didehydro-2′,3′-dideoxycytidin und Benzoesäureanhydrid in Pyridin das N-Benzoyl 2′,3′-didehydro-2′,3′-dideoxycytidin 5′-benzoat erhalten werden.

Beispiel 8

In Analogie zu Beispiel 1 kann aus 2′,3′-Didehydro-2′,3′-dideoxycytidin und Phenylessigsäureanhydrid in Pyridin das N-(Phenylacetyl) 2′,3′-didehydro-2′,3′-dideoxycytidin 5′-(phenylacetat) erhalten werden.

Beispiel 9

Eine Lösung von 8,00 g N-Acetyl 2′,3′-didehydro-2′,3′-dideoxycytidin 5′-acetat in 500 ml Methanol-Tetrahydrofuran (1:1) wurde über 0,10 g 10% Palladium/Kohle bei einer Atmosphäre Wasserstoffdruck hydriert. Die Lösung wurde durch Diatomeenerde filtriert und unter vermindertem Druck zur Trockene eingedampft. Der Rückstand wurde in 600 ml heissem Acetonitril gelöst, filtriert und auf 200 ml eingeengt. Das Gemisch wurde 1 Stunde auf 0° gekühlt und filtriert. In zwei Fraktionen wurden insgesamt 6,50 g N-Acetyl 2′,3′-dideoxycytidin 5′-acetat, Smp. 210-211°, $[\alpha]_D^{25}$ = +92° (c = 0,49, Methanol) erhalten.

Beispiel 10

In Analogie zu Beispiel 9 kann aus N-Propanoyl 2′,3′-didehydro-2′,3′-dideoxycytidin 5′-propanoat das N-Propanoyl 2′,3′-dideoxycytidin 5′-propanoat erhalten werden.

Beispiel 11

In Analogie zu Beispiel 9 kann aus N-(2-Methylpropanoyl)2′,3′-didehydro-2′,3′-dideoxycytidin 5′-(2-methylpropanoat) das N-(2-Methylpropanoyl) 2′,3′-dideoxycytidin 5′-(2-methylpropanoat) erhalten werden.

Beispiel 12

In Analogie zu Beispiel 9 kann aus N-Butanoyl 2′,3′-didehydro-2′,3′-dideoxycytidin 5′-butanoat das N-Butanoyl 2′,3′-dideoxycytidin 5′-butanoat erhalten werden.

Beispiel 13

In Analogie zu Beispiel 9 kann aus N-Palmitoyl 2′,3′-didehydro-2′,3′-dideoxycytidin 5′-palmitoat das N-Palmitoyl 2′,3′-dideoxycytidin 5′-palmitoat erhalten werden.

Beispiel 14

In Analogie zu Beispiel 9 kann aus N-Stearoyl 2′,3′-didehydro-2′,3′-dideoxycytidin 5′-stearat das N-Stearoyl 2′,3′-dideoxycytidin 5′-stearat erhalten werden.

Beispiel 15

In Analogie zu Beispiel 9 kann aus N-Benzoyl 2′,3′-didehydro-2′,3′-dideoxycytidin 5′-benzoat das N-Benzoyl 2′,3′-dideoxycytidin 5′-benzoat erhalten werden.

Beispiel 16

In Analogie zu Beispiel 9 kann aus N-(Phenylacetyl) 2′,3′-didehydro-2′,3′-dideoxycytidin 5′-(phenylacetat) das N-(Phenylacetyl) 2′,3′-didehydro-2′,3′-dideoxycytidin 5′-(phenylacetat) erhalten werden.

Beispiel 17

Ein Gemisch von 0,021 g 2′,3′-Dideoxycytidin, 0,25 ml Acetanhydrid und 1,0 ml trockenem Pyridin wurde 3 Stunden bei 25° gerührt. Das homogene Gemische wurde dann mit 5 ml Methanol verdünnt und eine halbe Stunde bei 25° gerührt. Das Gemisch wurde unter vermindertem Druck zur Trockene eingedampft und der Rückstand aus 1,0 ml Acetonitril umkristallisiert, wobei 0,024 g N-Acetyl 2′,3′-dideoxycytidin 5′-acetat, Smp. 210-211°, $[\alpha]_D^{25}$ = +91° (c = 0,50, Methanol) erhalten wurden.

Beispiel 18

Ein Gemisch von 10,0 g 2′,3′-Dideoxycytidin, 25,0 g Propionsäureanhydrid und 50 ml trockenem Pyridin wurde 18 Stunden bei Raumtemperatur gerührt. Die homogene Lösung wurde dann unter vermindertem Druck zur Trockene eingedampft. Der Rückstand wurde in 100 ml Aethylacetat gelöst, durch Diatomeenerde filtriert und das Filtrat unter vermindertem Druck zur Trockene eingedampft. Der Feststoff wurde mit vier 100 ml Portionen Hexan behandelt und getrocknet und lieferte 8,8 g N-Propanoyl 2′,3′-dideoxycytidin 5′-propionat, Smp. 110-112°, $[\alpha]_D^{15}$ = +83° (c = 0,59, Methanol). Dieser Feststoff bildete Gele mit verschiedenen Lösungsmitteln.

Beispiel 19

Ein Gemisch von 9,50 g 2′,3′-Dideoxycytidin, 30,0 g 2-Methylpropionsäureanhydrid und 60 ml trockenem Pyridin wurde 18 Stunden bei Raumtemperatur gerührt. Die homogene Lösung wurde unter vermindertem Druck zur Trockene eingedampft. Der Rückstand wurde in 300 ml heissem Aether gelöst, filtriert und unter vermindertem Druck zur Trockene eingedampft. Der Rückstand wurde in 150 ml heissem Aether gelöst und mit 150 ml Hexan versetzt. Nach Kühlen auf Raumtemperatur bildete sich ein dickes Gel. Das Gel wurde abfiltriert und unter vermindertem Druck getrocknet und lieferte 6,60 g N-(2-Methylpropanoyl) 2′,3′-dideoxycytidin 5′-(2-methylpropanoat), Smp. 120-122°, $[\alpha]_D^{25}$ = +86° (c = 0,60, Methanol).

Beispiel 20

Ein Gemisch von 10,5 g 2′,3′-Dideoxycytidin, 35,0 g Butansäureanhydrid und 70 ml trockenes Pyridin wurde 18 Stunden bei Raumtemperatur gerührt. Die homogene Lösung wurde dann unter vermindertem Druck zur Trockene eingedampft. Der Rückstand wurde in 350 ml heissem Aethylacetat gelöst, filtriert und unter vermindertem Druck zur Trockene eingedampft. Der Rückstand wurde dann mit 200 ml heissem Aether und 200 ml heissem Hexan verrieben und getrocknet und lieferte 8,55 g N-Butanoyl 2′,3′-dideoxcycytidin 5′-butanoat, Smp. 98-99°, $[\alpha]_D^{25}$ = +82° (c = 0,53, Methanol).

Beispiel 21

Ein Gemisch von 0,021 g 2′,3′-Dideoxycytidin, 0,25 ml Palmitoylchlorid und 1 ml Pyridin wurde 2 Stunden bei 0° gerührt. Das Gemisch wurde mit einigen Eisstücken versetzt und 10 Minuten weitergerührt. Das Gemisch wurde dann mit 75 ml Methylenchlorid verdünnt, die Lösung mit zwei 25 ml-Portionen

gesättigter wässriger Natriumbicarbonatlösung gewaschen, die wässrigen Phasen mit 25 ml Methylenchlorid rückextrahiert und die vereinigten organischen Phasen über Magnesiumsulfat getrocknet, filtriert und unter vermindertem Druck zur Trockene eingedampft. Der Rückstand wurde aus einer minimalen Menge heissem Methanol umkristallisiert und lieferte 0,058 g N-Palmitoyl 2′,3′-dideoxycytidin 5′-palmitat, Smp. 86-89°, $[\alpha]_D^{25}$ = +44° (c = 0,48, Methanol).

## Beispiel 22

In Analogie zu Beispiel 20 kann aus 2′,3′-Dideoxycytidin und Stearoylchlorid in Pyridin das N-Stearoyl 2′,3′-dideoxycytidin 5′-stearat erhalten werden.

## Beispiel 23

In Analogie zu Beispiel 21 kann aus 2′,3′-Dideoxycytidin und Benzoesäureanhydrid in Pyridin das N-Benzoyl 2′,3′-dideoxycytidin 5′-benzoat erhalten werden.

## Beispiel 24

In Analogie zu Beispiel 20 oder 21 kann aus 2′,3′-Dideoxycytidin und Phenylacetylchlorid oder Phenylacetanhydrid in Pyridin das N-(Phenylacetyl) 2′,3′-dideoxycytidin 5′-(phenylacetat) erhalten werden.

## Beispiel 25

Ein Gemisch von 21,1 g 2′,3′-Dideoxycytidin, 30,0 g Acetanhydrid und 1 l absolutem Aethanol wurde 1 Stunde bei 70-75° gerührt. Die homogene Lösung wurde unter vermindertem Druck zur Trockene eingedampft und der Rückstand mit 100 ml Toluol versetzt und unter vermindertem Druck zur Trockene abgedampft. Man erhielt 25,3 g N-Acetyl 2′,3′-dideoxycytidin, Smp. 140°, $[\alpha]_D^{25}$ = +103° (c = 0.56, Methanol).

## Beispiel 26

Ein Gemisch von 10 g 2′,3′-Dideoxycytidin, 1,36 g Propionsäureanhydrid und 500 ml absolutem Aethanol wurde 2 Stunden bei 65-70° gerührt. Die homogene Lösung wurde unter vermindertem Druck zur Trockene eingedampft. Der Rückstand wurde in 200 ml absolutem Aethanol gelöst, filtriert und unter vermindertem Druck zur Trockene eingedampft. Der Rückstand wurde dann aus 100 ml heissem absoluten Aethanol umkristallisiert und lieferte in zwei Fraktionen 5 g N-Propanoyl 2′,3′-dideoxycytidin, Smp.167-169°, $[\alpha]_D^{25}$ = +97° (c = 0,50, Methanol).

## Beispiel 27

Ein Gemisch von 9,50 g 2′,3′-Dideoxycytidin, 15,8 g 2-Methylpropionsäureanhydrid und 500 ml absolutem Aethanol wurde 1 Stunde bei 76-70° gerührt. Die homogene Lösung wurde unter vermindertem Druck zur Trockene eingedampft. Der Rückstand wurde in 200 ml Aethanol gelöst, die Lösung filtriert und zur Trockene eingedampft. Der Feststoff wurde aus 100 ml heissem absolutem Aethanol umkristallisiert und lieferte in zwei Fraktionen 8,50 g eines 1:1-Gemischs von N-(2-Methylpropanoyl) 2′,3′-dideoxycytidin und 2-Methylpropionsäure, Smp. 112-113°, $[\alpha]_D^{25}$ = +69° (c = 0,50, Methanol).

Eine Probe von 3 g wurden in 500 ml heissem Aethylacetat gelöst und die Lösung mit 200 ml gesättigter wässriger Natriumbicarbonatlösung gewaschen. Die wässrige Phase wurde mit 200 ml Aethylacetat rückextrahiert. Die vereinigten organischen Phasen wurden über Magnesiumsulfat getrocknet, filtriert und eingedampft. Man erhielt 1,50 g N-(2-Methylpropanoyl) 2′,3′-dideoxycytidin, Smp. 58-62°, $[\alpha]_D^{25}$ = +84° (c = 0,48, Methanol). Dieser Feststoff bildete mit verschiedenen Lösungsmitteln Gele.

## Beispiel 28

Ein Gemisch von 9,50 g 2′,3′-Dideoxycytidin, 15,8 g Butansäureanhydrid und 500 ml absolutem Aethanol wurde 1 Stunde bei 65-70° gerührt. Die homogene Lösung wurde unter vermindertem Druck zur Trockene eingedampft, der Feststoff in 200 ml absolutem Aethanol gelöst, filtriert und auf 100 ml zur Kristallisation eingeengt. Eine erste Fraktion bestand aus 6,80 g eines 2:1-Gemisches von N-Butanoyl 2′,3′-dideoxycytidin und Butansäure, Smp. 146-147°, $[\alpha]_D^{25}$ = +78° (c = 0,53, Methanol).

Eine 6,60 g-Probe wurde in 500 ml heissem Aethylacetat gelöst und die Lösung mit 100 ml gesättigter wässriger Natriumbicarbonatlösung gewaschen. Die wässrige Phase wurde mit 100 ml Aethylacetat rückextrahiert. Die vereinigten organischen Phasen wurden über Magnesiumsulfat getrocknet, filtriert und eingedampft und lieferten 4,50 g N-Butanoyl 2′,3′-dideoxycytidin, Smp. 154-156°, $[\alpha]_D^{25}$ = °88° (c = 0,56, Methanol). Dieser Feststoff bildete mit verschiedenen Lösungsmitteln Gele.

## Beispiel 29

In Analogie zu Beispiel 28 kann aus 2′,3′-Dideoxycytidin und Palmitinsäure in absolutem Aethanol N-Palmitoyl 2′,3′-dideoxycytidin erhalten werden.

## Beispiel30

In Analogie zu Beispiel 28 kann aus 2′,3′-Dideoxycytidin und Stearinsäureanhydrid in absolutem Aethanol das N-Stearoyl 2′,3′-dideoxycytidin erhalten werden.

## Beispiel 31

Ein Gemisch von 1 g 2′,3′-Dideoxycytidin, 2,3 g Benzoesäureanhydrid und 100 ml absolutem Aethanol wurde 18 Stunden zum Rückfluss erhitzt. Das Gemisch wurde gekühlt und unter vermindertem Druck zur Trockene eingedampft. Der Rückstand wurde mit 100 ml Aether behandelt, um Aethylbenzoat-Verunreinigungen zu entfernen. Der Rückstand wurde aus 140 ml Aethylacetat umkristallisiert und lieferte N-Benzoyl 2′,3′-dideoxycytidin, Smp. 174-175°, $[\alpha]_D^{25}$ = +90° (c = 0,50, Methanol).

## Beispiel 32

In Analogie zu Beispiel 28 kann aus 2′,3′-Dideoxycytidin und Phenylessigsäureanhydrid in absolutem Aethanol das N-(Phenylacetyl) 2′,3′-dideoxycytidin erhalten werden.

## Beispiel 33

Ein Gemisch von 0,5 g N-Acetyl 2′,3′-dideoxycytidin 5′-acetat und 100 ml 16%igem methanolischem Ammoniak wurde 1 Stunde bei Raumtemperatur gerührt. Das Gemisch wurde zur Trockene eingedampft.

Der Rückstand wurde durch Silicagelchromatographie gereinigt. Elution mit Methylenchlorid-Methanol (10:1) lieferte in den ersten Fraktionen 0,06 g N-Acetyl 2′,3′-dideoxycytidin, Smp. 140°, $[\alpha]_D^{25}$ = +103° (c = 0,56, Methanol). Die späteren Fraktionen lieferten 0,1 g 2′,3′-Dideoxycytidin 5′-acetat, Smp. 154-156°, $[\alpha]_D^{25}$ = +91° (c = 0,45, Methanol). Aehnliche Resultate wurden erhalten, wenn N-Acetyl 2′,3′-dideoxycytidin 5′-acetat mit einem Methanol-Wasser-Triäthylamingemisch (7:2:1) 1 Stunde bei Raumtemperatur behandelt wurde.

## Beispiel 34

In Analogie zu Beispiel 33 kann aus N-Propanoyl 2′,3′-dideoxycytidin 5′-propionat das N-Propanoyl 2′,3′-dideoxycytidin und das 2′,3′-Dideoxycytidin 5′-propanoat erhalten werden.

## Beispiel 35

Ein Gemisch von 3,4 g N-(2-Methylpropanoyl) 2′,3′-dideoxycytidin 5′-(2-methylpropanoat) und 150 ml 15%igem methanolischem Ammoniak wurde 1 Stunde bei Raumtemperatur gerührt und dann unter vermindertem Druck zur Trockene eingedampft. Der Rückstand wurde durch Säulenchromatogrpahie an Silicagel gereinigt. Elution mit Methylenchlorid-Methanol (25:1) lieferte N-(2-Methylpropanoyl) 2′,3′-dideoxycytidin, $[\alpha]_D^{25}$ = +79° (c = 0,50, Methanol). Elution mit Methylenchlorid-Methanol)15:1) lieferte dann 2′,3′-Dideoxycytidin 5′-(2-methylpropanoat), $[\alpha]_D^{25}$ = +67° (c = 0.54, Methanol).

## Beispiel 36

Ein Gemisch von 3,4 g N-Butanoyl 2′,3′-dideoxycytidin 5′-butanoat und 150 ml 15%igem methanolischem Ammoniak wurde 1 Stunde bei Raumtemperatur gerührt und dann unter vermindertem Druck zur Trockene eingedampft. Der Rückstand wurde durch Chromatographie an Silicagel gereinigt. Elution mit Methylenchlorid-Methanol (25:1) lieferte N-Butanoyl 2′,3′-dideoxycytidin. Elution mit Methylenchlorid-Methanol (15:1) lieferte 2′,3′-Dideoxycytidin 5′-butanoat, Smp. 99-103°, $[\alpha]_D^{25}$ = +76° (c = 0,54, Methanol).

## Beispiel 37

In Analogie zu Beispiel 36 kann aus N-Palmitoyl 2′,3′-dideoxycytidin 5′-palmitoat das N-Palmitoyl 2′,3′-dideoxycytidin und das 2′,3′-Dideoxycytidin 5′-palmitoat erhalten werden.

## Beispiel 38

In Analogie zu Beispiel 36 kann aus N-Stearoyl 2′,3′-dideoxycytidin 5′-stearat das N-Stearoyl 2′,3′-dideoxycytidin und das 2′,3′-Dideoxycytidin 5′-stearat erhalten werden.

## Beispiel 39

In Analogie zu Beispiel 36 kann aus N-Benzoyl 2′,3′-dideoxycytidin 5′-benzoat das N-Benzoyl 2′,3′-dideoxycytidin und das 2′,3′-Dideoxycytidin 5′-benzoat erhalten werden.

## Beispiel 40

In Analogie zu Beispiel 36 kann aus N-(Phenylacetyl) 2′,3′-dideoxycytidin 5′-(phenylacetat) das N-(Phenylacetyl) 2′,3′-dideoxycytidin und das 2′,3′-Dideoxycytidin 5′-(phenylacetat) erhalten werden.

Beispiel 41

Zu einer Lösung von 1,05 g 2′,3′-Dideoxycytidin in 5 ml Dimethylformamid wurden 2 ml N,N-Dimethylformamid-dimethylacetal gegeben. Nach 15 Stunden Rühren bei Raumtemperatur wurde das Reaktionsgemisch unter vermindertem Druck eingedampft und liefert 1,2 g 2′,3′-Dideoxy-N-[(dimethylamino)-methylen]cytidin als hellgelben Feststoff.

Beispiel 42

Ein Gemisch von 0,532 g 2′,3′-Dideoxy-N-[(dimethylamino)-methylen]cytidin, 3,0 ml Acetanhydrid und 4 ml Pyridin wurden 1 Stunde bei Raumtemperatur gerührt und lieferte 2′,3′-Dideoxy-N-[(dimethylamino)-methylen]cytidin 5′-acetat. Zu dieser Lösung wurden 5,0 ml Methanol gegeben und das Gemisch wurde unter vermindertem Druck zur Trockene eingedampft. Der Rückstand wurde mit 5 ml Toluol versetzt und unter vermindertem Druck zur Trockene eingedampft. Der Rückstand wurde dann hydrolysiert und durch Silicagelchromatographie gereinigt. Elution mit Methylenchlorid-Methanol (10:1) lieferte 1,05 g 2′,3′-Dideoxycytidin 5′-acetat, Smp. 154-156°, $[\alpha]_D^{25}$ = +91° (c = 0,45, Methanol).

Beispiel 43

In Analogie zu Beispiel 42 kann aus 2′,3′-Dideoxy-N-[(dimethylamino)methylen]cytidin und Propansäureanhydrid das 2′,3′-Dideoxy-N-[(dimethylamino)methylen]cytidin 5′-propionat und daraus das 2′,3′-Dideoxycytidin 5′-propionat erhalten werden.

Beispiel 44

In Analogie zu Beispiel 42 kann aus 2′,3′-Dideoxy-N-[(dimethylamino)methylen]cytidin und 2-Methylpropionsäureanhydrid das 2′,3′-Dideoxy-N-[(dimethlamino)methylen]cytidin 5′-(2-methylpropionat) und daraus das 2′,3′-Dideoxycytidin 5′-(2-methylpropionat) erhalten werden.

Beispiel 45

In Analogie zu Beispiel 42 kann aus 2′,3′-Dideoxy-N-[(dimethylamino)methylen]cytidin und Butansäureanhydrid das 2′,3′-Dideoxy-N-[(dimethylamino)methylen]cytidin 5′-butanoat und daraus das 2′,3′-Dideoxycytidin 5′-butanoat werden werden.

Beispiel 46

In Analogie zu Beispiel 42 kann aus 2′,3′-Dideoxy-N-[(dimethylamino)-methylen]cytidin und Palmitoylanhydrid das 2′,3′-Dideoxy-N-[(dimethylamino)methylen]cytidin 5′-palmitat und daraus das 2′,3′-Dideoxycytidin 5′-palmitat erhalten werden.

## Beispiel 47

In Analogie zu Beispiel 42 kann aus 2′,3′-Dideoxy-N-[(dimethlamino)methylen]cytidin und Stearoylanhydrid das 2′,3′-Dideoxy-N-[(dimethylamino)methylen]cytidin 5′-stearat und daraus das 2′,3′-Dideoxycytidin 5′-stearat erhalten werden.

## Beispiel 48

In Analogie zu Beispiel 42 kann aus 2′,3′-Dideoxy-N-[(dimethylamino)methylen]cytidin und Benzoesäureanhydrid das 2′,3′-Dideoxy-N-[(dimethylamino)methylen]cytidin 5′-benzoat und daraus das 2′,3′-Dideoxycytidin 5′-benzoat erhalten werden.

## Beispiel 49

In Analogie zu Beispiel 42 kann aus 2′,3′-Dideoxycytidin-N-[(dimethylamino)methylen]cytidin und Phenylessigsäureanhydrid das 2′,3′-Dideoxy-N-[(dimethylamino)methylen]cytidin 5′-(phenylacetat) und daraus das 2′,3′-Dideoxycytidin 5′-(phenylacetat) erhalten werden.

## Beispiel 50

Ein Gemisch von 2,09 g 2′,3′Didehydro-2′,3′-dideoxycytidin, 2,38 g N,N-Dimethylformamid-dimethylacetal und 4 ml N,N-Dimethylformamid wurde 18 Stunden bei Raumtemperatur gerührt. Das Gemisch wurde bei 40-45° /0,6 mbar zur Trockene eingedampft. Der Rückstand kristallisierte beim Stehen. Umkristallisation aus Methylenchlorid-Hexan lieferte 2′,3′-Didehydro-2′,3′-dideoxy -N-[(dimethylamino)methylen]cytidin, Smp. 150-151°, $[\alpha]_D^{25}$ = +50,8° (c = 0,534, Methanol).

Bei Behandlung der Substanz mit einem Anhydrid in Pyridin können in Analogie zu Beispiel 42 die folgenden Verbindungen hergestellt werden:

| | |
|---|---|
| Acetanhydrid | 2′,3′-Didehydro-2′,3′dideoxy-N-[(dimethylamino)methylen]cytidin 5′-acetat |
| Propionsäureanhydrid | 2′,3′-Didehydro-2′,3′-dideoxy-N-[(dimethylamino)methylen]cytidin 5′-propanoat |
| 2-Methylpropionsäureanhydrid | 2′,3′-Didehydro-2′,3′-dideoxy-N-[(dimethylamino)methylen]cytidin 5′-(2-methylpropanoat) |
| Buttersäureanhydrid | 2′,3′-Didehydro-2′,3′-dideoxy-N-[(dimethylamino)methylen]cytidin 5′-butanoat |
| Palmitinsäureanhydrid | 2′,3′-Didehydro-2′,3′-dideoxy-N-[(dimethylamino)methylen]cytidin 5′-palmitoat |
| Stearinsäureanhydrid | 2′,3′-Didehydro-2′,3′-dideoxy-N-[(dimethylamino)methylen]cytidin 5′-stearat |
| Benzoesäureanhydrid | 2′,3′-Didehydro-2′,3′-dideoxy-N-[(dimethylamino)methylen]cytidin 5′-benzoat |
| Phenylessigsäureanhydrid | 2′,3′-Didehydro-2′,3′-dideoxy-N-[(dimethylamino)methylen]cytidin 5′-(phenylacetat) |

Durch Behandlung dieser Verbindungen mit Silicagel erhält man die entsprechenden 2′,3′-Didehydro-2′,3′-dideoxycytidin 5′-ester.

## Beispiel 51

Ein Gemisch von 2,09 g 2′,3′-didehydro-2′,3′-dideoxycytidin, 5,1 g Acetanhydrid und 50 ml absolutem Aethanol wurde über Nacht bei Raumtemperatur gerührt. Die homogene Lösung wurde unter vermindertem Druck zur Trockene eingedampft. Der Feststoff wurde mit Aether verrieben und lieferte 2,2 g N-Acetyl 2′,3′-didehydro-2′,3′-dideoxycytidin, Smp. >350°.

Beispiel 52

Ein Gemisch von 2,09 g 2′,3′-Didehydro-2′,3′-dideoxycytidin, 6,5 g Propionsäureanhydrid und 50 ml absolutem Aethanol wurde über Nacht bei Raumtemperatur gerührt. Die homogene Lösung wurde dann unter vermindertem Druck zur Trockene eingeengt. Der Feststoff wurde mit Aether-Hexan verrieben und lieferte 2,2 g N-Propanoyl 2′,3′-didehydro-2′,3′-dideoxycytidin, Smp. 168-170° (Erweichung), 300° (Zersetzung), $[\alpha]_D^{25}$ = +101° (c = 0,59, Methanol).

Beispiel 53

Eine Gemisch von 2,09 g 2′,3′-Didehydro-2′,3′-dideoxycytidin, 7,91 g 2-Methylpropionsäureanhydrid und 50 ml absolutem Aethanol wurde über Nacht bei Raumtemperatur gerührt. Die homogene Lösung wurde zur Trockene eingedampft. Der Rückstand wurde mit Aether verrieben und lieferte 2,1 g N-(2-Methylpropanoyl) 2′,3′-didehydro-2′,3′-dideoxycytidin, Smp. 68-70° (Erweichung), 285° (Zersetzung), $[\alpha]_D^{25}$ = +78° (c = 0,57, Methanol).

Beispiel 54

Ein Gemisch von 2,09 g 2′,3′-Didehydro-2′,3′-dideoxycytidin, 7,91 g Butansäureanhydrid und 50 ml absolutem Aethanol wurde über Nacht bei Raumtemperatur gerührt. Die homogene Lösung wurde zur Trockene eingedampft. Der Rückstand wurde mit Aether verrieben und lieferte 2,4 g N-Butanoyl 2′,3′-didehydro-2′,3′-dideoxycytidin, Smp. 112-115° (Erweichung), 320° (Zersetzung), $[\alpha]_D^{25}$ = +82° (c = 0.47, Methanol).

Beispiel 55

In Analogie zu Beispiel 54 kann aus 2′,3′-Didehydro-2′,3′-dideoxycytidin und Palmitinsäureanhydrid das N-Palmitoyl 2′,3′-didehydro-2′,3′-dideoxycytidin erhalten werden.

Beispiel 56

In Analogie zu Beispiel 54 kann aus 2′,3′-Didehydro-2′,3′-dideoxycytidin und Stearinsäureanhydrid das N-Stearoyl 2′,3′-didehydro-2′,3′-dideoxycytidin erhalten werden.

Beispiel 57

In Analogie zu Beispiel 54 kann aus 2′,3′-Didehydro-2′,3′-dideoxycytidin und Benzoesäureanhydrid das N-Benzoyl 2′,3′-didehydro-2′,3′-dideoxycytidin erhalten werden.

Beispiel 58

In Analogie zu Beispiel 54 kann aus 2′,3′-Didehydro-2′,3′-dideoxycytidin und Phenylessigsäureanhydrid das N-Benzoyl 2′,3′-didehydro-2′,3′-dideoxycytidin erhalten werden.

Beispiel 59

Tabletten können mit konventionellen Verfahren aus folgenden Bestandteilen hergestellt werden:

| A. Bestandteil | mg/Tablette |
|---|---|
| 1. Verbindung der Formel I | 0.10 |
| 2. Lactose, wasserfrei | 149.25 |
| 3. Eisenoxid, rot | 0.15 |
| 4. Mikrokristalline Cellulose (Avicel PH-102) | 40.0 |
| 5. Croscarmellose Natrium, Typ A | 8.40 |
| 6. Magnesiumstearat | 2.10 |
| Total | 200.00 mg |

| B. Bestandteil | mg/Tablette |
|---|---|
| 1. Verbindung der Formel I | 5.00 |
| 2. Lactose, wasserfrei | 144.20 |
| 3. FD&C Blue #1 Lake | 0.10 |
| 4. Mikrokristalline Cellulose (Avicel PH-102) | 40.0 |
| 5. Croscarmellose Natrium, Typ A | 8.40 |
| 6. Magnesiumstearat | 2.30 |
| Total | 200.00 mg |

Beispiel 60

Sterile Pulver für Injektionszwecke können mit konventionellen Lyophilisationsverfahren aus folgenden Bestandteilen hergestellt werden:

| Bestandteil | mg/Ampulle |
|---|---|
| 1. Verbindung der Formel I | 10.00 |
| 2. Mannit | 50.00 |
| 3. Salzsäure (1% v/v) | |
| 4. Wasser für Injektionszwecke | |
| 1. Das pH der Grundlösung wird mit HCl eingestellt.<br>2. Die Lösung wird lyophilisiert. | |

## Ansprüche

1. Verbindungen der Formel

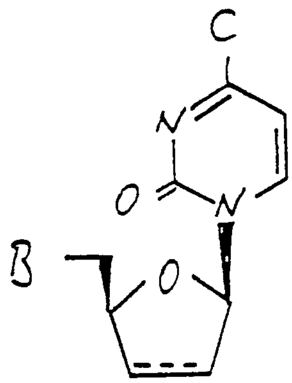

I

worin die punktierte Bindung fakultativ ist, B OH oder OC(O)R; C NHC(O)$R^1$ oder N=CH-N$R^2R^3$; R, $R^1$, $R^2$ und $R^3$ unabhängig voneinander geradkettiges oder verzweigtes Alkyl, Alkoxyalkyl, Aralkyl, Aryloxyalkyl oder Aryl darstellen.

2. Verbindungen gemäss Anspruch 1, in denen die punktierte Bindung in Formel I abwesend ist.

3. Verbindungen gemäss Anspruch 1 oder 2, in denen B OC(O)R und R Methyl, Aethyl, Propyl, Isopropyl, n-Pentadecyl, n-Heptydecyl, Phenyl oder Benzyl darstellt.

4. Verbindungen gemäss Anspruch 3, in denen C NHC(O)$R^1$ und $R^1$ Methyl, Aethyl, Propyl, Isopropyl, n-Pentadecyl, n-Heptadecyl, Phenyl oder Benzyl darstellen.

5. Verbindungen gemäss Anspruch 2, in denen B OH ist.

6. N-Butanoyl-2′,3′-dideoxycytidin.

7. N-Acetyl 2′,3′-dideoxycytidin;
N-Propanoyl 2′,3′-dideoxycytidin;
N-(2-Methylpropanoyl) 2′,3′-dideoxycytidin;
N-Palmitoyl 2′,3′-dideoxycytidin;
N-Stearoyl 2′,3′-dideoxycytidin;
N-Benzoyl 2′,3′-dideoxycytidin;
N-(Phenylacetyl) 2′,3′-dideoxycytidin;
2′,3′-Dideoxy-N-[(dimethylamino)methylen]cytidin;
N-Acetyl 2′,3′-dideoxycytidin 5′-acetat;
N-Propanoyl 2′,3′-dideoxycytidin 5′-propanoat;
N-(2-Methylpropanoyl) 2′,3′-dideoxycytidin 5′-(2-methylpropanoat);
N-Butanoyl 2′,3′-dideoxycytidin 5′-butanoat;
N-Palmitoyl 2′,3′-dideoxycytidin 5′-palmitoat;
N-Stearoyl 2′,3′-dideoxycytidin 5′-stearat;
N-Benzoyl 2′,3′-dideoxycytidine 5′-benzoate;
N-(Phenylacetyl) 2′,3′-dideoxycytidin 5′-(phenylacetat);
2′,3′-Dideoxy-N-[(dimethylamino)methylen]cytidin 5′-butanoat;
2′,3′-Dideoxy-N-[(dimethylamino)methylen]cytidin 5′-palmitat;
2′,3′-Dideoxy-N-[(dimethylamino)methylen]cytidin 5′-stearat;
2′,3′-Dideoxy-N-[(dimethylamino)methylen]cytidin 5′-benzoat;
2′,3′-Dideoxy-N-[(dimethylamino)methylen]cytidin 5′-acetat;
2′,3′-Dideoxy-N-[(dimethylamino)methylen]cytidin 5′-propanoat;
2′,3′-Dideoxy-N-[(dimethylamino)methylen]cytidin 5′-(2-methylpropanoat);
2′,3′-Dideoxy-N-[(dimethylamino)methylen]cytidin 5′-(phenylacetat).

8. N-Acetyl 2′,3′-didehydro-2′,3′-dideoxycytidin;
N-(2-Methylpropanoyl) 2′,3′-didehydro-2′,3′-dideoxycytidin;
N-Propanoyl 2′,3′-didehydro-2′,3′-dideoxycytidin;
N-Palmitoyl 2′,3′-didehydro-2′,3′-dideoxycytidin;
N-Stearoyl 2′,3′-didehydro-2′,3′-dideoxycytidin;
N-Butanoyl 2′,3′-didehydro-2′,3′-dideoxycytidin;

N-Benzoyl 2',3'-didehydro-2',3'-dideoxycytidin;
N-(Phenylacetyl) 2',3'-didehydro-2',3'-dideoxycytidin;
N-Acetyl-2',3'-didehydro-2',3'-dideoxycytidin 5'-acetat;
N-Propanoyl 2',3'-didehydro-2',3'-dideoxycytidin 5'-propanoat;
N-(2-Methylpropanoyl) 2',3'-didehydro-2',3'-dideoxycytidin 5'-(2-methylpropanoat);
N-Butanoyl2',3'-didehydro-2',3'-dideoxycyidin 5'-butanoat;
N-Palmitoyl 2',3'-didehydro-2',3'-dideoxycytidin 5'-palmitoat;
N-Stearoyl 2',3'-didehydro-2',3'-dideoxycytidin 5'-stearat;
N-Benzoyl 2',3'-didehydro-2',3'-dideoxycytidin 5'-benzoat;
N-(Phenylacetyl) 2',3'-didehydro-2',3'-dideoxycytidin 5'-(phenylacetat);
2',3'-Didehydro-2',3'-dideoxy-N-[(dimethylamino)methylen]cytidin;
2',3'-Didehydro-2',3'-dideoxy-N-[(dimethylamino)methylen]cytidin 5'-acetat;
2',3'-Didehydro-2',3'-dideoxy-N-[(dimethylamino)methylen]cytidin 5'-propanoat;
2',3'-Didehydro-2',3'-dideoxy-N-[(dimethylamino)methylen]cytidin 5'-(2-methylpropanoat);
2',3'-Didehydro-2',3'-dideoxy-N-[(dimethylamino)methylen]cytidin 5'-butanoat;
2',3'-Didehydro-2',3'-dideoxy-N-[(dimethylamino)methylen]cytidin 5'-(phenylacetat);
2',3'-Didehydro-2',3'-dideoxy-N-[(dimethylamino)methylen]cytidin 5'-palmitat;
2',3'-Didehydro-2',3'-dideoxy-N-[(dimethylamino)methylen]cytidin 5'-stearat;
2',3'-Didehydro-2',3'-dideoxy-N-[(dimethylamino)methylen]cytidin 5'-benzoat.

9. Die Verbindungen von Anspruch 1-8 als Heilmittel.

10. Verfahren zur Herstellung der Verbindung gemäss Anspruch 1, dadurch gekennzeichnet, dass man
a) eine Verbindung der Formel

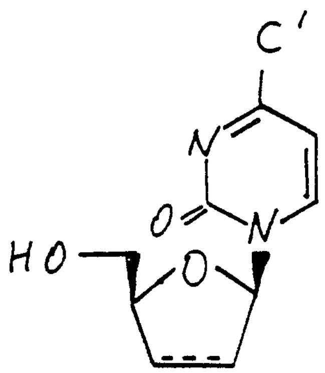

II

worin die punktierete Bindung fakultativ ist und C' $NH_2$ oder $N=CH-NR^2,R^3$ darstellt,
mit einem Anhydrid der Formel $(RCO)_2O$ oder einem Halogenid der Formel RCOX, worin R die obige Bedeutung hat und X Halogen ist, umsetzt oder
b) eine Verbindung der Formel II, worin C' $NH_2$ ist, mit einer Verbindung der Formel

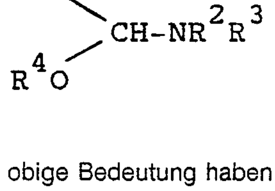

worin $R^4$ nieder-Alkyl und $R^2$ und $R^3$ die obige Bedeutung haben,
umsetzt und gewünschtenfalls die in einer erhaltenen Verbindung der Formel I vorhandene punktierte Bindung hydriert und/oder eine Estergruppe B oder eine Acylamido oder Schiff-Base-Gruppe C in einer erhaltenen Verbindung der Formel I selektiv hydrolysiert.

11. Pharmazeutische Präparate, enthaltend eine Verbindung der Ansprüche 1-8 und einen pharmazeutischen Träger.

12. Die Verbindungen der Ansprüche 1-8 zur Verwendung als Wirkstoffe bei der Herstellung von Heilmitteln gegen retrovirale Infektionen, insbesondere Aids.

VERBINDUNG 1a

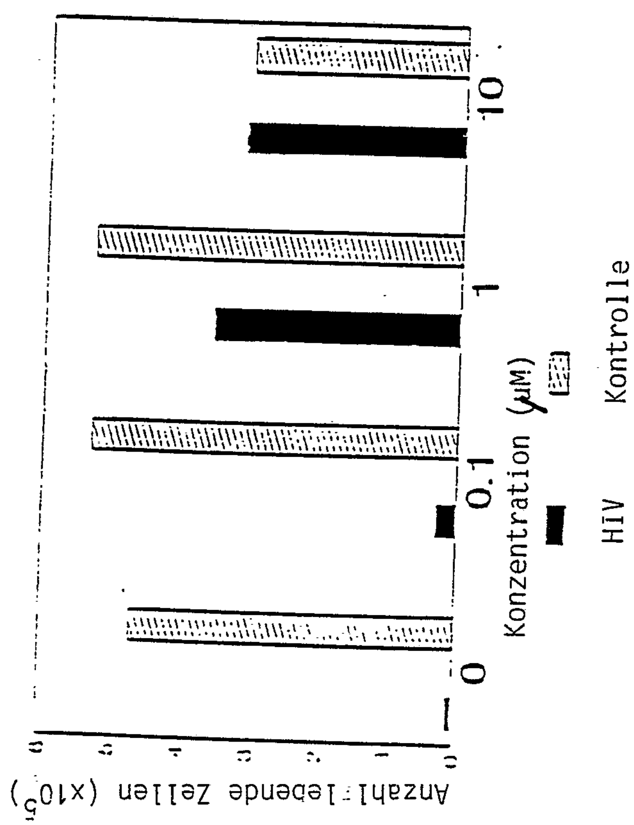


Figur 1

VERBINDUNG 2a

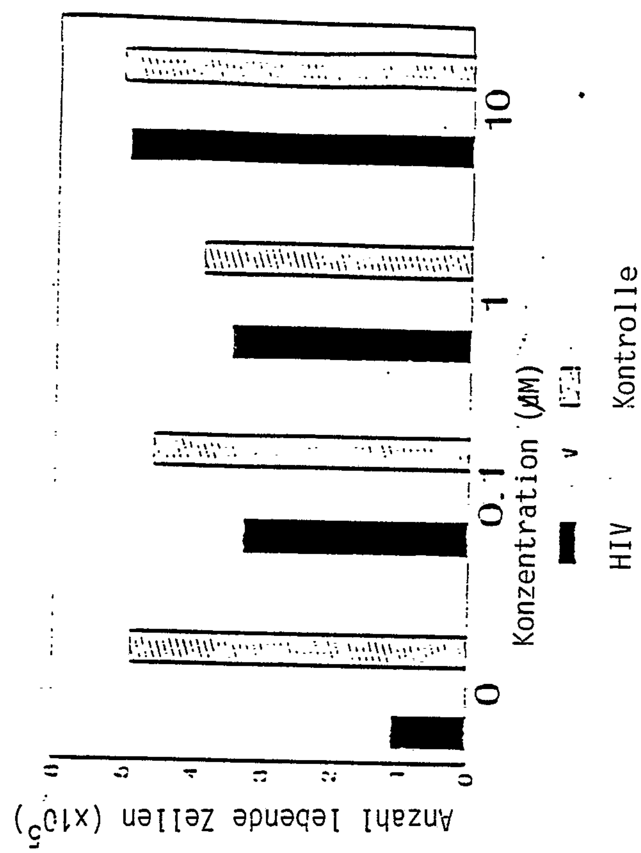


Figur 2

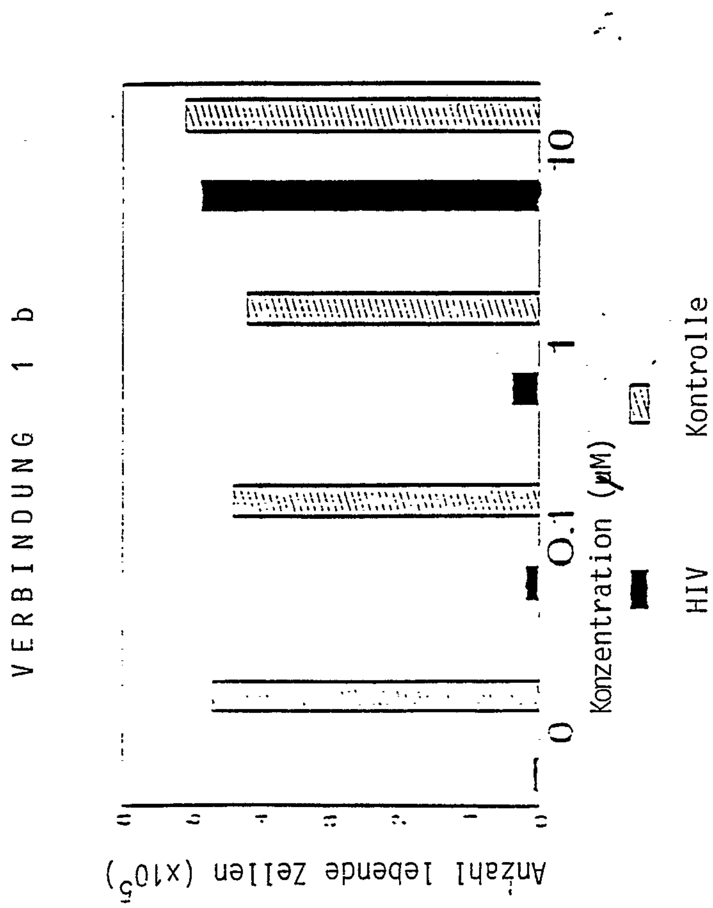


Figur 3

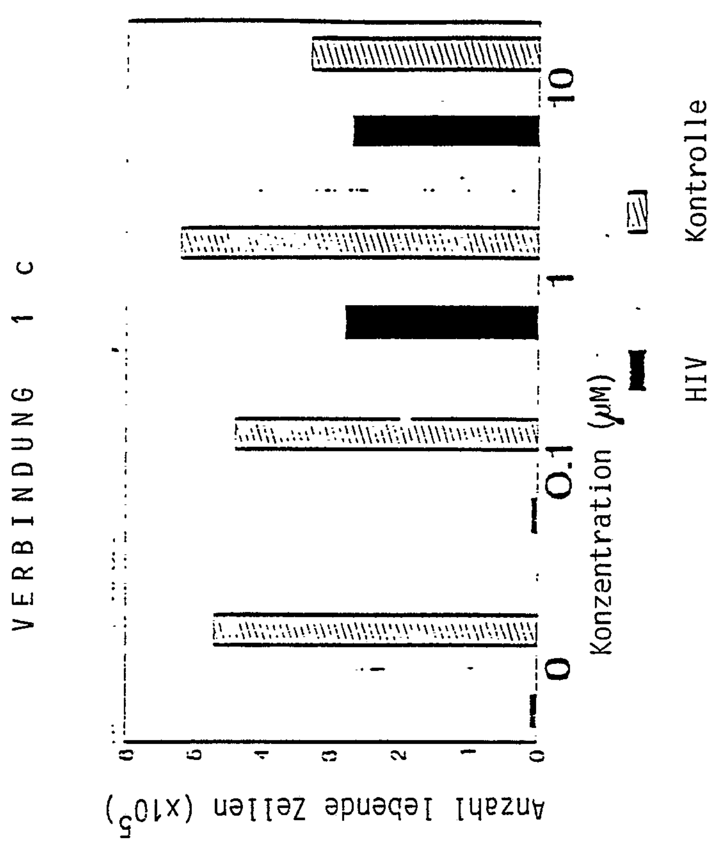


Figur 4

VERBINDUNG 2c

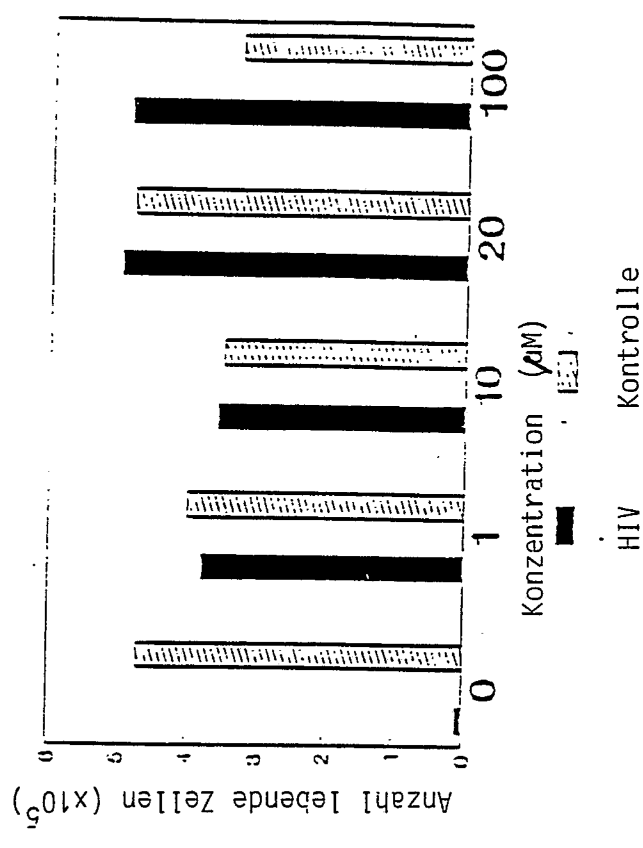


Figur 5